(19) Europäisches Patentamt
European Patent Office
Office européen des brevets

(11) Veröffentlichungsnummer: **0 325 784**
**A2**

## (12) EUROPÄISCHE PATENTANMELDUNG

(21) Anmeldenummer: **88121614.7**

(22) Anmeldetag: **23.12.88**

(51) Int. Cl.4: **C07C 45/00 , C07C 49/255 , C07C 49/76 , C07C 4/04 , C07C 49/203**

(30) Priorität: **31.12.87 DE 3744619**

(43) Veröffentlichungstag der Anmeldung:
**02.08.89 Patentblatt 89/31**

(84) Benannte Vertragsstaaten:
**BE CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Sproesser, Linhard, Dr.**
**Goethestrasse 4**
**D-6702 Bad Duerkheim(DE)**
Erfinder: **Sperling, Karin, Dr.**
**Waldstrasse 112**
**D-6706 Wachenheim(DE)**
Erfinder: **Trautmann, Walter, Dr.**
**Ritterbueschel 87**
**D-6730 Neustadt(DE)**
Erfinder: **Smuda, Hubert, Dr.**
**Rahmengasse 32**
**D-6900 Heidelberg(DE)**

## (54) Verfahren zur Herstellung von Ketonen.

(57) Verfahren zur Herstellung von Ketonen aus Säurehalogeniden mit Grignardverbindungen in inerten Lösungsmitteln, dadurch gekennzeichnet, daß man den Grignardverbindungen vor der Umsetzung mit Carbonsäurehalogeniden eine oder mehrere Verbindungen der allgemeinen Formeln Ia, Ib oder Ic

$Li_2MnX_4$ (Ia)
$LiFeX_4$ (Ib) oder
$Li_2CuX_4$ (Ic)

zusetzt, in denen X für Fluor, Chlor, Brom oder Iod steht.

EP 0 325 784 A2

## Verfahren zur Herstellung von Ketonen

Die vorliegende Erfindung betrifft ein Verfahren zur Herstellung von symmetrischen und unsymmetrischen Ketonen durch Umsetzung von Säurehalogeniden mit Grignardverbindungen in inerten Lösungsmitteln, indem man den Grignardverbindungen vor der Umsetzung mit Carbonsäurehalogeniden eine oder mehrere Verbindungen der allgemeinen Formeln Ia, Ib oder Ic

$Li_2MnX_4$ (Ia),
$LiFeX_4$ (Ib) oder
$Li_2CuX_4$ (Ic)

zusetzt, in denen X für Fluor, Chlor, Brom oder Iod steht.

Es ist bekannt, daß bei der Umsetzung von Carbonsäurechloriden mit Grignardreagentien die Ketone in der Regel nur in schlechten bis mäßigen Ausbeuten isoliert werden können, da die gebildeten Ketone mit dem Carbonsäurechlorid um Grignard-Reagens konkurrieren und die Bildung von Folgeprodukten wie Hydroxiverbindungen nicht verhindert werden kann.

Aus Houben-Weyl, Methoden der Organischen Chemie, Bd. 7/2a, S. 558ff, G. Thieme-Verlag, Stuttgart, (1973) ist bekannt, daß man bessere Ausbeuten an Ketonen erzielt, wenn man das Grignard-Reagens zunächst zur Quecksilber-, Cadmium-, Zink- oder Aluminium-alkylverbindung umsetzt und diese mit dem Carbonsäurechlorid reagieren läßt. Diese Methoden haben aber die Nachteile, daß sich z.B. die Quecksilber-, Cadmium-, Zink-, bzw. Aluminium-tert.-alkylverbindungen nur schlecht bilden und sie sich bei Temperaturen oberhalb 0 °C zersetzen. Außerdem müssen Cadmium, Quecksilber, Zink oder Aluminium in stöchiometrischen Mengen eingesetzt werden; darüber hinaus handelt es sich bei Quecksilber, Cadmium und Zink um stark toxische Übergangsmetalle.

Bessere Ausbeuten an unsymmetrischen oder symmetrischen Ketonen erhält man, wenn man Lithiumalkylkupferreagentien einsetzt. Diese Reagentien werden in drei- bis sechsfacher stöchiometrischer Menge eingesetzt. Diese Methoden haben Nachteile, weil sie teure Lithiumalkylverbindungen in großen Überschüssen benötigen. Zudem werden sehr tiefe Temperaturen (-20 bis -78 °C) für die Umsetzungen empfohlen [Houben-Weyl, Meth. d. Organischen Chemie, Bd. 7/2a, S. 580f, Stuttgart (1973); Normant et al. Tetrahedron Lett. (1976), 3155; Bourjain, Normant, Bull, Soc. Chim, Fr. (1973), 2137 und G. H. Posner, Org. Reactions 22, 253 (1975)].

Weiterhin ist bekannt, daß Salzzusätze die Ausbeuten an Ketonen erhöhen, z.B. kann man Grignard-Reagentien zusammen mit äquivalenten Mengen Mangan-II-jodid in Ether dem umzusetzenden Säurechlorid zusetzen (Normant et al., Synthesis 1977, 130 ff.). Diese Verfahrensweise hat allerdings den Nachteil, daß äquivalente und stöchiometrische Mengen eines teuren Jodids eingesetzt werden müssen.

Außerdem werden Salzzusätze von Übergangsmetallchloriden zur Grignardlösung empfohlen, wie z.B. $MnCl_2$, $FeCl_3$ oder Cu/CuCl-Mischungen (Houben-Weyl, Meth. d. Org. Chemie, Bd, 7/2a, S, 578 f., 1973). Diese Methoden ergeben stark schwankende Ausbeuten und schlechte Ergebnisse.

Ferner sind aus Modern Synthetic Methods, Vol. 3, Seite 139-216 (1983) R. Scheffold, John Wiley and Sons Kupfer- und Mangansalze in Grignardreaktionen als äquimolare Reagentien bekannt, wie das $Li_2MnCl_4$, das mit n-Butylmagnesiumchlorid in äquimolaren Mengen verwendet bei der Umsetzung mit n-Buttersäurechlorid zum Di-n-Butylketon, eingesetzt wurde.

Der Erfindung lag daher die Aufgabe zugrunde, einen verbesserten Zugang zu symmetrisch und unsymmetrisch substituierten Ketonen zu finden und den Nachteilen der bekannten Verfahren abzuhelfen.

Demgemäß wurde ein neues und verbessertes Verfahren zur Herstellung von Ketonen durch Umsetzung von Carbonsäurehalogeniden mit Grignardverbindungen in inerten Lösungsmitteln gefunden, wobei man den Grignardverbindungen vor der Umsetzung mit Säurehalogeniden eine oder mehrere Verbindungen der allgemeinen Formeln Ia, Ib oder Ic

$Li_2MnX_4$ (Ia),
$LiFeX_4$ (Ib) oder
$Li_2CuX_4$ (Ic)

zusetzt, in denen X für Fluor, Chlor, Brom oder Iod steht.

Die Umsetzung kann wie folgt durchgeführt werden:

Das Carbonsäurehalogenid wird in einem inerten organischen Lösungsmittel vorgelegt und eine Lösung des Grignard-Reagens und einer katalytischen Menge Ia, Ib und/oder Ic in einem organischen Lösungsmittel zugetropft.

Die Umsetzungen können zwischen (-78) bis +20 °C durchgeführt werden, vorzugsweise bei Temperaturen zwischen 0 und +20 °C.

Das Verfahren kann kontinuierlich oder diskontinuierlich durchgeführt werden.

Die Verbindungen Ia, Ib und Ic sind Addukte und können leicht durch Auflösen von Mangan(II-)chlorid, Eisen(III)-chorid oder Kupfer(II)-chlorid und Lithiumchlorid in aprotischen organischen Lösungsmittel gebildet werden. Diese Lösungen haben eine definierte Stöchiometrie und sind ohne Wirkungsverlust über längere Zeiträume, z.B. ein halbes Jahr, stabil.

Die Carbonsäurechloride und Grignard-Reagentien sind bekannt oder nach allgemeinen Standardmethoden (vgl. Organikum, 11. Aufl., S. 555 und S. 467) herstellbar.

Das Molverhältnis Gignard-Reagenz:Carbonsäurechlorid beträgt von 10:1 bis 0,01:1, bevorzugt von 5:1 bis 0,8:1 besonders bevorzugt 3:1 bis 1:1.

Die Verbindungen Ia, Ib und/oder Ic können in 0,0001 bis 10 Mol.%, vorzugsweise 0,001 bis 10 Mol.%, bezogen auf die Grignardverbindung verwendet werden. Besonders bevorzugt verwendet man 0,01 bis 5 Mol.% der Verbindung Ib bzw. 1 bis 6 Mol.% der Verbindung Ic bezogen auf die eingesetzte Grignardverbindung.

Die Verwendung von 1000 ml bis 2000 ml eines inerten organischen Lösungsmittels pro Mol Grignardreagens empfiehlt sich in aller Regel.

Als inerte organische Lösungsmittel eignen sich dipolare, aprotische organische Lösungsmittel, z.B. Ether, wie Diethylether, Methyl-tert.-butylether, Tetrahydrofuran, Dioxan und Dimethylglykolether; oder Amine, wie Triethylamin, oder apolare, aprotische organische Lösungsmittel, z.B. aliphatische Kohlenwasserstoffe, wie Cyclohexan oder aromatische Kohlenwasserstoffe, wie Benzol, Toluol als Gemisch mit dipolaren, aprotischen Lösungsmitteln. Besonders bevorzugt verwendet man Tetrahydrofuran als Lösungsmittel.

Unter den Verbindungen Ia, Ib und Ic eignen sich solche, die im Falle von Ia aus 2 LiX und $MnX_2$ im Falle von Ib aus LiX und $FeX_3$ und im Falle von Ic aus 2 LiX und $CuX_2$ zusammengesetzt sind. Der Rest X bedeutet Fluor, Chlor, Brom und Iod und kann in den einzelnen Salzkomponenten unterschiedlich sein, so daß die Addukte auch mehrere unterschiedliche Halogenide tragen können. Bevorzugt sind für die Verbindungen Ia $Li_2MnCl_4$ und $Li_2MnBr_2Cl_2$ für die Verbindungen Ib $LiFeCl_4$, $LiFeBr_4$, $LiFeBrCl_3$ und für die Verbindungen Ic $Li_2CuCl_4$, $Li_2CuBr_4$, $Li_2CuBr_2Cl_2$.

Besonders bevorzugt sind $Li_2MnCl_4$, $LiFeCl_4$ und $Li_2CuCl_4$.

Es eignen sich alle für Grignardreaktionen anwendbaren Carbonsäurehalogenide. Unter den Carbonsäurehalogeniden sind die Chloride und Bromide bevorzugt, besonders bevorzugt sind die Chloride.

Die Carbonsäurehalogenide haben folgende allgemeine Formel:

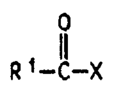

X = Fluor, Chlor, Brom, Iod.

Der Rest $R^1$ hat bevorzugt folgende Bedeutung:

- unverzweigtes und verzweigtes $C_1$-$C_{30}$-Alkyl, bevorzugt unverzweigtes oder verzweigtes $C_1$-$C_{16}$-Alkyl, besonders bevorzugt unverzweigtes oder verzweigtes $C_1$-$C_{12}$-Alkyl, wie Methyl, Ethyl, n-Propyl, iso-Propyl, n-Butyl, iso-Butyl, sec.-Butyl, tert.-Butyl, n-Pentyl, n-Hexyl, n-Octyl, n-Nonyl, n-Decyl,
- unverzweigtes oder verzweigtes $C_2$-$C_{30}$-Alkenyl, bevorzugt unverzweigtes oder verzweigtes $C_2$-$C_{16}$-Alkenyl, wie Vinyl, Allyl, 1-Methylvinyl, 2-Methylvinyl, 2,2-Dimethylvinyl, 1-Methylallyl, 1,1-Dimethylallyl, 2-Methylallyl, 3,3-Dimethylallyl, 2-Ethyl-but-1-en-1-yl, E-Hexadec-8-en-1-yl, Z-Hexadec-8-en-1-yl, E/Z-Hexadec-8-en-1-yl,
- unverzweigtes oder verzweigtes $C_3$-$C_{30}$-Alkinyl mit nicht endständiger Dreifachbindung, bevorzugt $C_3$-$C_{20}$-Alkinyl mit nicht endständiger Dreifachbindung, besonders bevorzugt $C_3$-$C_{12}$-Alkinyl mit nicht endständiger Dreifachbindung, wie Prop-1-inyl,
- $C_3$-$C_{20}$-Cycloalkyl, bevorzugt $C_3$-$C_{12}$-Cycloalkyl, besonders bevorzugt $C_3$-$C_8$-Cycloalkyl, wie Cyclopropyl, Cyclobutyl, Cyclopentyl, Cyclohexyl, Cycloheptyl und Cyclooctyl,
- $C_4$-$C_{20}$-Alkylcycloalkyl, bevorzugt $C_4$-$C_{12}$-Alkylcycloalkyl, wie 2,2-Dimethylcyclopropyl,
- isolierte oder konjugierte $C_3$-$C_{20}$-Cycloalkenyl, bevorzugt isolierte oder konjugierte $C_3$-$C_{12}$-Cycloalkenyl, besonders bevorzugt isolierte oder konjugierte $C_3$-$C_8$-Cycloalkenyl, wie Cycloprop-1-en-1-yl, Cycloprop-2-en-1-yl, Cyclobut-1-en-1-yl, Cyclobut-2-en-1-yl, Cyclopent-1-en-1-yl, Cyclopent-2-en-1-yl, Cyclopent-3-en-1-yl, Cyclohex-1-en-1-yl, Cyclohex-2-en-1-yl, Cyclohex-3-en-1-yl, Cyclohept-1-en-1-yl, Cyclohept-2-en-1-yl, Cyclohept-3-en-1-yl, Cyclohept-4-en-1-yl, Cyclooct-1-en-1-yl, Cyclooct-2-en-1-yl, Cyclooct-3-en-1-yl, Cyclooct-4-en-1-yl, Cyclobutadienyl, 1,3-Cyclopentadien-1-yl, 2,4-Cyclopentadien-1-yl, 1,3-Cyclohexadien-1-yl, 1,4-Cyclohexadien-1-yl, 2,4-Cyclohexadien-1-yl, 2,5-Cyclohexadien-1-yl,
- Aryl, bevorzugt Phenyl, 1-Naphthyl, 2-Naphthyl, 1-Anthracenyl, 2-Anthracenyl und 9-Anthracenyl, besonders bevorzugt Phenyl,
- Hetaryl, bevorzugt ein, zwei oder drei Heteroatome aus der Gruppe Sauerstoff, Schwefel und/oder Stickstoff enthaltende $C_3$-$C_{12}$-Hetaryl, besonders bevorzugt ein, zwei oder drei Heteroatome aus der Gruppe Sauerstoff, Schwefel und/oder Stickstoff enthaltende $C_3$-$C_8$-Hetaryl, wie 2-Furanyl, 3-Furanyl, 2-Thiophenyl, 3-Thiophenyl, 1-Pyrrolyl, 2-Pyrrolyl, 3-Pyrrolyl, 2-Pyridyl, 3-Pyridyl,
- durch ein, zwei oder drei Heteroatome aus der Gruppe Sauerstoff, Schwefel und/oder Stickstoff enthaltende $C_3$-$C_{20}$-Cycloalkyl, bevorzugt durch ein, zwei oder drei Heteroatome aus der Gruppe Sauerstoff, Schwefel und/oder Stickstoff enthaltende $C_3$-$C_{12}$-Cycloalkyl, besonders bevorzugt durch ein, zwei oder drei Heteroatome aus der Gruppe Sauerstoff, Schwefel und/oder Stickstoff enthaltende $C_3$-$C_8$-Cycloalkyl,
- $C_2$-$C_{30}$-Alkoxyalkyl, bevorzugt $C_2$-$C_{16}$-Alkoxyalkyl, besonders bevorzugt $C_2$-$C_8$-Alkoxyalkyl,
- $C_7$-$C_{30}$-Arylalkyl, bevorzugt $C_7$-$C_{20}$-Phenylalkyl, besonders bevorzugt $C_7$-$C_{16}$-Phenylalkyl,
- $C_7$-$C_{30}$-Arylalkoxy, bevorzugt $C_7$-$C_{20}$-Phenylalkoxy, besonders bevorzugt $C_7$-$C_{16}$-Phenylalkoxy,
- $C_2$-$C_{30}$-Alkylaminoalkyl, bevorzugt $C_2$-$C_{20}$-Alkylaminoalkyl, besonders bevorzugt $C_2$-$C_{16}$-Alkylaminoalkyl,
- $C_7$-$C_{30}$-Arylaminoalkyl, bevorzugt
- $C_2$-$C_{30}$-Alkoxycarbonyl, bevorzugt $C_2$-$C_{20}$-Alkoxycarbonyl, besonders bevorzugt $C_2$-$C_{12}$-Alkoxycarbonyl, Ethoxycarbonylmethyl,
- $C_2$-$C_{30}$-Halogencarbonylalkyl, bevorzugt $C_2$-$C_{20}$-Halogencarbonylalkyl, besonders bevorzugt $C_2$-$C_{12}$-Fluor-, Chlor- oder Brom-carbonylalkyl, Chlorcarbonyl-n-butyl,
- Diphenyl, wie o-Diphenyl, m-Diphenyl und p-Diphenyl,
- durch ein, zwei oder drei Halogenatome substituiertes Phenyl, wie 2-Fluorphenyl, 3-Fluorphenyl, 4-Fluorphenyl, 2-Chlorphenyl, 3-Chlorphenyl, 4-Chlorphenyl, 2-Bromphenyl, 3-Bromphenyl, 4-Bromphenyl, 2,3-Difluorphenyl, 2,4-Difluorphenyl, 2,5-Difluorphenyl, 2,6-Difluorphenyl, 3,4-Difluorphenyl, 3,5-Difluorphenyl, 2,3-Difluorphenyl, 2,4-Dichlorphenyl, 2,5-Dichlorphenyl, 2,6-Dichlorphenyl, 3,4-Dichlorphenyl, 3,5-Dichlorphenyl, 2,3-Dibromphenyl, 2,4-Dibromphenyl, 2,5-Dibromphenyl, 2,6-Dibromphenyl, 3,4-Dibromphenyl, 3,5-Dibromphenyl, 2-Chlor-3-fluorphenyl, 3-Chlor-2-fluorphenyl, 2-Chlor-4-fluorphenyl, 4-Chlor-2-fluorphenyl, 2-Chlor-5-fluorphenyl, 5-Chlor-2-fluorphenyl, 2-Chlor-6-fluorphenyl, 3-Chlor-4-fluorphenyl, 4-Chlor-3-fluorphenyl, 3-Chlor-5-fluorphenyl, 2-Brom-3-fluorphenyl, 3-Brom-2-fluorphenyl, 2-Brom-4-fluorphenyl, 4-Brom-2-fluorphenyl, 2-Brom-5-fluorphenyl, 5-Brom-2-fluorphenyl, 2-Brom-6-fluorphenyl, 3-Brom-4-fluorphenyl, 4-Brom-3-fluorphenyl, 3-Brom-5-fluorphenyl, 2-Brom-3-chlorphenyl, 3-Brom-2-chlorphenyl, 2-Brom-4-chlorphenyl, 4-Brom-2-chlorphenyl, 2-Brom-5-fluorphenyl, 5-Brom-2-chlorphenyl, 4-Brom-6-chlorphenyl, 3-Brom-4-chlorphenyl, 4-Brom-3-chlorphenyl, 3-Brom-5-chlorphenyl,
- durch ein, zwei oder drei $C_1$-$C_4$-Alkylgruppen substituiertes Phenyl,
- durch ein, zwei oder drei $C_1$-$C_4$-Alkoxygruppen substituiertes Phenyl,
- durch ein, zwei oder drei $C_1$-$C_4$-Halogenalkylgruppen substituiertes Phenyl,
- durch ein oder zwei Nitrogruppen substituiertes Phenyl,
- durch ein, zwei oder drei Phenoxygruppen substituiertes Phenyl,
- durch Halogen und $C_1$-$C_4$-Alkyl zwei- oder dreifach substituiertes Phenyl,
- durch Halogen und $C_1$-$C_4$-Alkoxy zwei- oder dreifach substituiertes Phenyl,
- durch Halogen und $C_1$-$C_4$-Halogenalkyl zwei- oder dreifach substituiertes Phenyl,
- durch Halogen und Nitro zwei- oder dreifach substituiertes Phenyl,
- durch Halogen und Phenoxy zwei- oder dreifach substituiertes Phenyl,
- durch $C_1$-$C_4$-Alkyl und $C_1$-$C_4$-Alkoxy zwei- oder dreifach substituiertes Phenyl,
- durch $C_1$-$C_4$-Alkyl und $C_1$-$C_4$-Halogenalkyl zwei- oder dreifach substituiertes Phenyl,
- durch $C_1$-$C_4$-Alkyl und Nitro zwei- oder dreifach substituiertes Phenyl,
- durch $C_1$-$C_4$-Alkyl und Phenoxy zwei- oder dreifach substituiertes Phenyl,
- durch $C_1$-$C_4$-Alkoxy und $C_1$-$C_4$-Halogenalkyl zwei- oder dreifach substituiertes Phenyl,
- durch $C_1$-$C_4$-Alkoxy und Nitro zwei- oder dreifach substituiertes Phenyl,
- durch $C_1$-$C_4$-Alkoxy und Phenoxy zwei- oder dreifach substituiertes Phenyl,
- durch $C_1$-$C_4$-Halogenalkyl und Nitro zwei- oder dreifach substituiertes Phenyl,
- durch $C_1$-$C_4$-Halogenalkyl und Phenoxy zwei- oder dreifach substituiertes Phenyl,
- durch Nitro und Phenoxy zwei- oder dreifach substituiertes Phenyl,
- durch Halogen, $C_1$-$C_4$-Alkyl und $C_1$-$C_4$-Alkoxy dreifach substituiertes Phenyl,
- durch Halogen, $C_1$-$C_4$-Alkyl und $C_1$-$C_4$-Halogenalkyl dreifach substituiertes Phenyl
- durch Halogen, $C_1$-$C_4$-Alkyl und Nitro dreifach substituiertes Phenyl
- durch Halogen, $C_1$-$C_4$-Alkyl und Phenoxy dreifach substituiertes Phenyl
- durch Halogen, $C_1$-$C_4$-Alkoxy und $C_1$-$C_4$-Halogenalkyl dreifach substituiertes Phenyl
- durch Halogen, $C_1$-$C_4$-Alkoxy und Nitro dreifach substituiertes Phenyl
- durch Halogen, $C_1$-$C_4$-Alkoxy und Phenoxy dreifach substituiertes Phenyl

- durch Halogen, $C_1$-$C_4$-Halogenalkyl und Nitro dreifach substituiertes Phenyl
- durch Halogen, $C_1$-$C_4$-Halogenalkyl und Phenoxy dreifach substituiertes Phenyl
- durch Halogen, Nitro und Phenoxy dreifach substituiertes Phenyl
- durch $C_1$-$C_4$-Alkyl, $C_1$-$C_4$-Alkoxy und $C_1$-$C_4$-Halogenalkyl dreifach substituiertes Phenyl
- durch $C_1$-$C_4$-Alkyl, $C_1$-$C_4$-Alkoxy und Nitro dreifach substituiertes Phenyl
- durch $C_1$-$C_4$-Alkyl, $C_1$-$C_4$-Alkoxy und Phenoxy dreifach substituiertes Phenyl
- durch $C_1$-$C_4$-Alkyl, $C_1$-$C_4$-Halogenalkyl und Nitro dreifach substituiertes Phenyl
- durch $C_1$-$C_4$-Alkyl, $C_1$-$C_4$-Halogenalkyl und Phenoxy dreifach substituiertes Phenyl
- durch $C_1$-$C_4$-Alkyl, Nitro und Phenoxy dreifach substituiertes Phenyl
- durch $C_1$-$C_4$-Alkoxy, $C_1$-$C_4$-Halogenalkyl und Nitro dreifach substituiertes Phenyl
- durch $C_1$-$C_4$-Alkoxy, $C_1$-$C_4$-Halogenalkyl und Phenoxy dreifach substituiertes Phenyl
- durch $C_1$-$C_4$-Halogenalkyl, Nitro und Phenoxy dreifach substituiertes Phenyl.

Als Grignardreagentien eignen sich alle herstellbaren Grignardverbindungen
$R^2$-Mg-Y    Y = Fluor, Chlor, Brom, Iod.

Unter den Grignardreagentien sind solche bevorzugt, in denen Y für Chlor und Brom steht.

Der Rest $R^2$ hat bevorzugt folgende Bedeutung:
- unverzweigtes oder verzweigtes $C_1$-$C_{30}$-Alkyl, bevorzugt unverzweigtes oder verzweigtes $C_1$-$C_{20}$-Alkyl, besonders bevorzugt unverzweigtes oder verzweigtes $C_1$-$C_{12}$-Alkyl, wie Methyl, Ethyl, n-Propyl, iso-Propyl, n-Butyl, iso-Butyl, sec.-Butyl, tert.-Butyl, n-Pentyl, iso-Pentyl, n-Hexyl, n-Heptyl, n-Octyl, n-Nonyl, n-Decyl, N-Dodecyl.

Die nach dem erfindungsgemäßen Verfahren hergestellten symmetrischen und unsymmetrischen Ketone sind wertvolle Zwischenprodukte für Wirkstoffe, die auf dem Gebiet des Pflanzenschutzes Verwendung finden (DE-A-30 19 049, EP-A-150 404).

## Beispiele

Herstellung von tert.-Butylmagnesiumchlorid-Lösung, diskontinuierlich siehe Whitmore et al., J. Am. Chem. Soc. 54, 3716 (1932) und Whitmore et al., J. Am. Chem. Soc. 55, 1652 (1933) sowie EP 0150 404.

Eine acidimetrische Gehaltsbestimmung einer kleinen Probe ergab eine Grignard-Konzentration von 1,2 mol/l.

Herstellung von tert.-Butylmagnesiumchlorid-Lösung, kontinuierlich:

In einer Röhren-Umlaufapparatur, die mit einem Glassieb, einem Thermometer, einem Kühler, einem Zulauf, einem Überlauf und einem Umpumpmotor bestückt war, wurden 500 g Magnesiumspäne eingefüllt. Nach dem Einfüllen von 2,2 l Tetrahydrofuran (THF), was dem Betriebsvolumen entsprach, wurde das Lösungsmittel im Kreis umgepumpt und mittels eines an den Kühler angeschlossenen Thermostaten auf 30 °C temperiert. Die Apparatur wurde mit Stickstoff inertisiert. Dann wurden kontinuierlich 300 ml/h einer 1,5 molaren Lösung von tert.-Butylchlorid in Tetrahydrofuran zugepumpt. Nachdem die Konzentrationen einen stationärem Zustand erreicht hatten, wurde in der am Überlauf entnommenen Lösung eine Konzentration von 1,2 mol/l Grignard-Reagens festgestellt.

Herstellung von Methylmagnesiumchlorid-Lösung:

siehe Coburn, Org. Synth. Coll. Vol. III (1955), S. 696

Herstellung der Katalysatorlösungen:

Lithiumtetrachloromanganat-(II)

In einem Liter Tetrahydrofuran wurden 42,3 g (1,0 mol) Lithiumchlorid und 63 g (0,5 mol) Mangan(II)-chlorid unter Feuchtigkeitsausschluß gerührt, bis sich alles gelöst hatte.

Lithiumtetrachloroferrat-(III)

In einem Liter Tetrahydrofuran wurden 21,2 g (0,5 mol) Lithiumchlorid und 81,1 g (0,5 mol) Eisen(III)-chlorid unter Feuchtigkeitsausschluß gerührt, bis sich alles gelöst hatte.

Lithiumtetrachlorocuprat-(II)

In einem Liter Tetrahydrofuran wurden 42,3 g (1,0 mol) Lithiumchlorid und 67,2 g (0,5 mol) Kupfer(II)-chlorid unter Feuchtigkeitsausschluß gerührt, bis sich alles gelöst hatte.

Beispiel 1

Herstellung von 2,2-Dimethyl-6-phenoxy-hexan-3-on, diskontinuierlich

Zu 150 ml der von Magnesiumresten befreiten und gerührten tert.-Butylmagnesiumchlorid-Lösung (s. o.) wurden unter $N_2$-Schutzgasatmosphäre bei +10 °C 10,5 ml der oben beschriebenen 0,5 molaren Lithiumtetrachloromanganat-(II)-lösung entsprechend 7 Mol-% getropft. Anschließend wurde eine Lösung von 14,9 g (0,075 mol) Phenoxybuttersäurechlorid in 40 ml Tetrahydrofuran zugetropft. Nach 20 min war die Umsetzung beendet. Nach Hydrolyse mit wässriger Ammoniumchloridlösung und extraktiver Aufarbeitung erhielt man nach dem Einengen 14 g rohes 2,2-Dimethyl-6-phenoxy-hexan-3-on, mit einem Gehalt von 75 % des gewünschten Ketons (Gaschromatographisch bestimmt), das durch Destillation weiter gereinigt werden konnte.

Beispiel 2

Herstellung von tert.-Butyrophenon

Untersucht wurde die Ausbeute an Keton in Abhängigkeit von der Katalysatorkonzentration. 50 ml einer 1,0 molaren tert.-Butylgrignardlösung wurden mit verschiedenen Mengen 0,5 molarer $Li_2MnCl_4$-Lösung bzw. 0,5 molarer $Li_2CuCl_4$-Lösung versetzt. Bei +10 °C wurden 3,5 g (0,025 mol) Benzoylchlorid in 10 ml Tetrahydrofuran zugetropft. Aufgearbeitet wurde durch Hydrolyse und Extraktion.

Die Ergebnisse sind in Tabelle 1 zusammengefaßt.

Tabelle 1

| Art des Katalysators | Stöchiometrische Mengen bezogen auf Grignard | Gehalt an Keton im Reaktions-Austrag |
|---|---|---|
| $Li_2CuCl_4$ | 10 Mol-% | 90 % |
| $Li_2CuCl_4$ | 1 Mol-% | 96 % |
| $Li_2CuCl_4$ | 0,1 Mol-% | 93 % |
| $Li_2CuCl_4$ | 0,01 Mol-% | 95 % |
| $Li_2CuCl_4$ | 0,001 Mol-% | 77 % |
| $Li_2CuCl_4$ | 0,0001 Mol-% | 77 % |
| $Li_2MnCl_4$ | 10 Mol-% | 88 % |
| $Li_2MnCl_4$ | 1 Mol-% | 81 % |
| $Li_2MnCl_4$ | 0,1 Mol-% | 83 % |
| $Li_2MnCl_4$ | 0,01 Mol-% | 82 % |
| $Li_2MnCl_4$ | 0,001 Mol-% | 81 % |
| $Li_2MnCl_4$ | 0,0001 Mol-% | 77 % |
| | Kontrollexperiment ohne Katalysator | 72 % |

Beispiel 3

Herstellung von 2,2-Dimethyl-6-phenoxy-hexan-3-on, diskontinuierlich, Katalysator $LiFeCl_4$

Analog zu Beispiel 1, Katalysator 10,5 ml $LiFeCl_4$-Lösung
Reaktionsdauer: 25 min
Ausbeute: 11,9 g (Gehalt 87 %, Nebenprodukt Phenoxybuttersäure-omega-Chlorbutylester)

Beispiel 4

Herstellung von 2,2-Dimethyl-6-phenoxy-hexan-3-on, diskontinuierlich, Katalysator $Li_2CuCl_4$

Analog Beispiel 1 mit 800 ml einer 1,2 molaren tert.-Butylmagnesiumchloridlösung, 200 ml 0,5 molarer $Li_2CuCl_4$-Lösung und 100 g (0,51 mol) Phenoxybuttersäurechlorid in 200 ml Tetrahydrofuran. Reaktionsdauer 18 min. Ausbeute 82 g roh, Gehalt 86 % (Gaschromatographisch).

Vergleichsexperiment:

99 g Phenoxybuttersäurechlorid wurden in 300 ml Tetrahydrofuran vorgelegt. Unter Rühren und $N_2$-Schutzgas wurden bei -10 °C 300 ml 1,2 molarer tert.-Butylmagnesiumchloridlösung zugetropft. Nach Hydrolyse und extraktiver Aufarbeitung mit Natronlauge zur Entfernung überschüssiger Phenoxybuttersäure erhielt man 48 g eines Rohprodukts, das 39 % 2,2-Dimethyl-6-phenoxy-hexan-3-on enthielt. Von den zahlreichen Nebenprodukten konnte nur die korrespondierende Hydroxyverbindung identifiziert werden.

Beispiel 5

Herstellung von 2,2-Dimethyl-6-phenoxy-hexan-3-on kontinuierlich

Mit der oben beschriebenen, kontinuierlichen Apparatur zur Herstellung von tert.-Butylmagnesiumchlorid wurden 300 ml Grignard-Lösung pro Stunde erzeugt. In einem ersten nachgeschalteten Reaktor wurden bei +5 °C bis +7 °C 30 ml/h 0,5 molarer Lithiumtetrachlorocuprat-Lösung in Tetrahydrofuran, entsprechend ca.

5 mol-% zudosiert. In einem zweiten Nachreaktor, der mit dem Überlauf des ersten Nachreaktors verbunden war, wurden bei 0 °C verschiedene Mengen einer 1-molaren Lösung von Phenoxybuttersäurechlorid in Tetrahydrofuran zudosiert. Die Mengen wurden von 150 ml/h bis 270 ml/h Zulauf variiert. Die Versuche wurden jeweils so lange durchgeführt, bis sich ca. 800 ml Rohaustrag angesammelt hatten. Die Aufarbeitung der Rohausträge geschah wie unter Beispiel 1) und 2) beschrieben.

Die Ergebnisse sind in Tabelle 2 zusammengefaßt.

Tabelle 2

| Zulauf Phenoxybuttersäurechlorid Lösung | Stöchiometr. Verhältnis Grignard/Säurechlorid | Gehalt Keton |
|---|---|---|
| a) 150 ml/h | 1 : 0,5 | 94,5 % |
| b) 180 ml/h | 1 : 0,6 | 86,7 % |
| c) 220 ml/h | 1 : 0,73 | 71,5 % |
| d) 240 ml/h | 1 : 0,8 | 69,5 % |
| e) 270 ml/h | 1 : 0,9 | 66,4 % |

Als Hauptnebenprodukte der Ansätze c), d) und e) wurden Phenoxybuttersäure-tert.-butylester und Phenoxybuttersäure-omega-chlor-n-butylester gefunden.

Beispiel 6

Herstellung von 2,2-Dimethyl-6-phenoxy-hexan-3-on, kontinuierlich

Der unter Beispiel 5a) beschriebene Versuch wurde mit 300 ml Zulauf/h Grignard-Reagens, 30 - 40 ml Zulauf/h Lithiumtetrachlorocuprat-II-lösung wiederholt. Die Konzentrationen wurden analog Beispiel 4) gewählt.

Nach 27 Stunden wurden nach Aufarbeitung 697 g Rohprodukt erhalten, Ausbeute 85 %.

Beispiel 7 - 15

Analog Beispiel 4 wurden mit 160 ml einer 1,2 molaren tert.-Butylmagnesiumchlorid-Lösung, 40 ml $Li_2CuCl_4$-Lösung und 0,1 mol Säurechlorid in 50 ml Tetrahydrofuran die folgenden Ketone hergestellt (Tabelle 3):

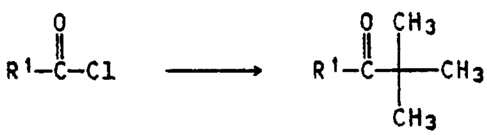

Tabelle 3

| Beispiel | $R^1$ | Ausbeute | |
|---|---|---|---|
| | | mit | ohne |
| | | Katalysator | |
| 7 | Methyl | 60 % | 10 % |
| 8 | tert-Butylcarbonyl-n-butyl | 71 % | - |
| 9 | n-Decyl | 82 % | 7 % |
| 10 | Isopropyl | 34 % | <2 % |
| 11 | 2-Ethylbut-1-en-1-yl | 84 % | 28 % |
| 12 | Z-Hexadec-8-en-1-yl | 87 % | 12 % |
| 13 | Ethoxycarbonylmethyl | 61 % | - |
| 14 | p-Chlorphenyl | 81 % | 41 % |
| 15 | 3-Pyridyl | 47 % | <2 % |

$^1$H-NMR-Spektroskopische Daten zu Beispiel 1 - 15

Beisp. $^1$H-NMR-chemische Verbindungen, δ-Werte bezogen auf TMS

1 1,2(s;9H);2,0(m;2H);2,6(t;2H);3,9(t;2H);6,9(m;3H);7,2(m;3H)
2 1,35 (s;9H); 7,3(m;3H);7,6(m;2H)
7 1,15(s;9H);2,1(s;3H)
8 1,15(s;18H);1,6(m;;4H);2,2(t;4H)
9 0,9(t;3H);1,18(s;9H);1,3(m;16H);2,2(t;2H)
10 0,9(q;3H);1,09(q;3H);1,2(s;9H);2,5(m;1H)
11 1,15(t;3H);1,45(t;3H);1,45(s;9H);1,95(q;2H);2,25(q;2H);5,6(s;1H)
12 0,9(t;3H);1,2(s;9H);1,25-1,4(m;20H);1,6(m;4H);2,1(t;2H);5,3(m;2H)
13 1,3(t;3H);1,5(s;9H);3,25(s;2H);4,2(q;2H)
14 1,2(s;9H);7,4(d;2H);7,75(d;2H)
15 1,2(s;9H);7,4(dd;2H);8,0(d;1H);8,25(dd;1H)

Beispiel 16

Herstellung von Pinakolon, Katalysator $Li_2CuCl_4$

Analog Beispiel 4 wurden 600 ml 1,5 molarer Methylmagnesiumchlorid-Lösung, 200 ml 0,5 molarer $Li_2CuCl_4$-Lösung und 60 g (0,5 mol) Pivalinsäurechlorid in 200 ml Tetrahydrofuran umgesetzt. Nach Hydrolyse, Extraktion und Destillation erhielt man 32,4 g Pinakolon der Reinheit 97 %.

Kontrollexperiment ohne Katalysator:

300 ml 1,5 molare Methylmagnesiumchlorid-Lösung (entsprechend 0,45 mol Reagens) wurden auf -10 °C abgekühlt. Dann wurden 60 g (0,5 mol) Pivalinsäurechlorid in 200 ml Tetrahydrofuran so zugesetzt, daß die Temperatur zwischen -10 °C und -12 °C blieb. Man erhielt nach Extraktion 23,2 g Rohprodukt, das zu 60 % aus Pinakolon und 30 % 3,3-Dimethyl-2-butanol bestand.

**Ansprüche**

1. Verfahren zur Herstellung von Ketonen durch Umsetzung von Säurehalogeniden mit Grignardverbindungen in inerten Lösungsmitteln, dadurch gekennzeichnet, daß man den Grignardverbindungen vor der Umsetzung mit Carbonsäurehalogeniden eine oder mehrere Verbindungen der allgemeinen Formeln Ia, Ib oder Ic

$Li_2MnX_4$ (Ia),

$LiFeX_4$ (Ib) oder

$Li_2CuX_4$ (Ic)

zusetzt, in denen X für Fluor, Chlor, Brom oder Iod steht.

2. Verfahren nach Anspruch 1, dadurch gekennzeichnet, daß man die Verbindungen Ia, Ib bzw. Ic in 0,0001 bis 10 Mol.% bezogen auf die Grignardverbindung verwendet.

3. Verfahren nach Anspruch 1, dadurch gekennzeichnet, daß man die Verbindungen Ia, Ib bzw. Ic in 0,001 bis 10 Mol.% bezogen auf die Grignardverbindung verwendet.

4. Verfahren nach Anspruch 1, dadurch gekennzeichnet, daß man die Verbindung Ib in 0,01 bis 5 Mol.% bezogen auf die Grignardverbindung verwendet.

5. Verfahren nach Anspruch 1, dadurch gekennzeichnet, daß man die Verbindung Ic in 1 bis 6 Mol.% bezogen auf die Grignardverbindung verwendet.

6. Verfahren nach Anspruch 1, dadurch gekennzeichnet, daß man Verbindungen der Formeln Ia, Ib oder Ic verwendet, in denen X für Chlor steht.